# EUROPEAN PATENT SPECIFICATION

(11) **EP 3 352 714 B1**
(45) Date of publication and mention of the grant of the patent: **20.03.2019**
(21) Application number: 16778457.8
(22) Date of filing: 14.09.2016
(51) Int. Cl.: A61F 5/01

(54) **SYSTEM FOR THE ADJUSTMENT AND TAUTENING OF THE FIXING STRAPS OF AN ORTHOSIS FOR JOINTS**
SYSTEM ZUR EINSTELLUNG UND STRAFFUNG DER FIXIERBÄNDER EINER ORTHESE FÜR GELENKE
SYSTÈME POUR RÉGLER ET TENDRE DES SANGLES DE FIXATION D'UNE ORTHÈSE POUR ARTICULATIONS

(30) Priority: 22.09.2015 IT UB20153806
(43) Date of publication of application: 01.08.2018
(73) Proprietor: F.G.P. S.R.L., 37062 Villafranca Di Verona (Verona) (IT)
(72) Inventor: TURRINI, Alberto, 37062 Villafranca di Verona (Verona) (IT); FERRIGOLO, Moreno, 37062 Villafranca di Verona (Verona) (IT)
(74) Representative: Sandri, Sandro
(86) International application number: PCT/IB2016/055459
(87) International publication number: WO 2017/051285

(56) References cited:
- US-A- 5 807 294
- US-A- 5 857 989
- US-A1- 2004 059 270
- US-A1- 2004 267 179
- US-A1- 2005 203 455

## Description

### TECHNICAL FIELD

This invention refers to a system for the adjustment and control of the lateral thrusts of an orthopedic brace for joints and in particular for the adjustment of the lateral thrust force transmitted to the joint by an appropriate support, duly connected to straps subjected to traction by means of two mobile elements, for a knee brace for osteoarthrosis or other similar orthopedic brace designed for the rehabilitation of joints of the human body, such as the joints of the elbow, the ankle, the shoulder, and the like.

This solution foresees the creation of a brace with features designed to correct certain problems of the joints of the knee, the elbow or of other joints, mainly connected to the problem of varus or valgus deformity.

In particular, the system for the adjustment and tensioning according to the invention foresees the use of sliding guides which allow the controlled movement of the attachment points of the traction straps along the uprights of just one of the two articulated couplings of the brace, in order to increase the distance between the attachment points of the straps positioned on the opposite articulated coupling, this variation in distance creating traction on one of the two condylar cushion pads, the thrust one, which is pulled towards the ideal centre of rotation of the joint in question, pushing on the condyles and realigning the skeletal axes in a correct position.

This invention can be applied in the medical and orthopedic industry and in particular in the production of braces in general, as well as of prostheses and orthoses mainly used in conservative, post-traumatic, re-educational and post-operative therapy.

### BACKGROUND ART

It is known that subjects presenting orthopedic problems involving the knee joint, but similarly also of other joints, such as the ankle or the elbow, above all in the case of injuries or as a result of a previous surgical operation, require the use of an orthopedic brace, or orthosis, designed to guarantee or control the hinge restraint function between the femur and the tibia or other articulated joint points in general, supporting stress which would otherwise be harmful for the joint.

The function of an orthosis is, in general, to guarantee the relative immobilization or limitation of a joint affected by trauma, by arthrosis, by sprained ligaments or which has undergone a surgical operation.

Another use of orthoses is for the concomitant functional rehabilitation or re-education, wherein the orthosis can be used to reduce the load on the joint and decrease the pain, or can be used for prevention in cases of osteoporosis or bone weakness.

An orthosis usually comprises a rigid or soft frame, encircling the limb, designed to guarantee adequate harnessing of the joint in order to prevent the onset of stress on the ligaments or on the synovial membranes during walking by the injured and/or convalescent subject.

Some types of orthoses are designed for the postural irregularities of subjects with a varus or valgus knee which, especially in older persons, can cause wear to the cartilage of the knee joints that may lead to acute or chronic inflammation of the joints or varying degrees of osteoarthritis, all characterised by pain which limits already impaired gait.

Braces for joints in general and for the knee joint in particular consist of a rigid frame designed to ensure adequate anchorage to the limb in order to avoid stress on the articulation and to limit or prevent torsion during walking.

Taking the knee joint into consideration, the frame of classic knee braces comprises means for constraining the brace to the femur and to the tibia in areas proximal to the knee and a structure connecting these means with a hinge positioned at the level of the knee.

To ensure that the limb has sufficient freedom of movement, the frame develops for a good distance laterally to the knee, in order to allow reciprocal oscillation between the femur and the tibia.

The means of constraint usually consist of straps fitted with Velcro, particularly suitable for adjustment of the locking tension, these straps being attached to appropriate fixed supports in order to wrap around the areas of the body forming part of the joint in question.

For persons presenting varus or valgus knee problems, the structures designed for normal alignment of the joint are often unable to fully satisfy the requirements of protection and appropriate therapeutic effect, and in addition are not particularly comfortable to wear.

While a structure specifically designed for this type of problem must be able to be wrapped around the joint, following the individual conformation of the limb, the production of structures made according to the requirements of individual patients is not a solution that can be proposed at industrial level.

Other solutions foresee the use of particular flexible hinges, inserted in the side arms of the structure and which make it possible to adjust the brace on the joint, whether it be a varus or valgus knee.

A sought-after effect of such braces is the ability to relieve the medial or lateral pain caused by the osteoarthritis, and in particular these braces attempt to provide a thrust to counter the thrust caused by the disorder, by applying a pressure that alters the contact between the opposite condyles of the knee bones, reducing the pressure on them and limiting the pain.

In some cases, the brace comprises a structure with a hinge operating at an angle in order to apply force on the joint.

This structure used screws for the adjustment of the angle of each hinge and thus of the force applied to the joint. The appropriate positioning of the arm of the structure, and the correct force to be applied on the joint involve a somewhat impractical operation that cannot be carried out independently and in an immediate way by the person wearing the brace.

In another case, such as the one patented by the same applicant under number B02015A000079, an articulated coupling for orthoses presents, with respect to known solutions, a biaxial multicentre type construction, with the two primary rotation centres of the bars fixed in turn on a single-centre system that thus eliminates the restraint of parallelism between the axes.

By introducing this new secondary rotation centre, the bars are able to rotate around the primary centre of rotation and around this new common centre, thus making it possible to create lateral thrusts, specifically designed to correct valgism or varism problems.

A circular grooved cursor, which slides along the axis of the secondary rotation centre, allows the bars to remain connected to each other even when they rotate around the secondary rotation centre, becoming in fact detached from the original rotation centre.

Close to the inner side of each of the secondary rotation centres is a thrust cushion pad that can exert controlled pressure on the condylar parts of the joint to be rehabilitated, in order to reduce the pressure on the affected area, that is to say the area opposite the thrust.

These solutions foresee that the force of the lateral thrusts is applied close to the rotation axes of the secondary rotation centres, making it difficult to exert sufficient force to correctly impart the necessary thrusts.

In fact, in this working condition, the thrusts exerted on the condylar cushion pads must be increased in order to reach the required level, placing the brace under stress which in the long term could impair the stability of the structure.

Document US-A-5807294 discloses an orthosis having the features described in the preamble of claim 1.

Furthermore, document US-A-5857989 discloses a dynamic orthopedic knee brace assembly restricting anterior tibial movement. The assembly includes a proximal cuff for engaging the wearer's leg above the knee and a distal cuff for engaging the wearer's leg below the knee. The proximal and distal cuffs are linked together by a hinge that permits pivotal movement of the proximal cuff relative to the distal cuff. The proximal cuff has lateral and medial portions each having a slot extending there along. A strap guiding assembly is slidably mounted within each of the slots. Each strap guiding assembly is arranged to slide between a proximal extreme when the wearer's leg is in flexion and a distal extreme as the wearer extends his or her leg. A biasing device biases each strap guiding asssmbly towards its proximal extreme.

### DESCRIPTION OF THE INVENTION

This invention proposes to provide a system for the adjustment and tensioning of the fixing straps of a knee brace for osteoarthrosis or other similar orthopedic brace designed for the rehabilitation of joints of the human body, that can eliminate or at least reduce the problems described above.

The invention proposes in particular to provide a system for the adjustment and tensioning of the fixing straps of a knee brace for osteoarthrosis or other similar orthopedic brace, that foresees the controlled movement of the thrusts using the straps that hold the brace in place.

It has in fact been observed that the efficacy of braces equipped with this new secondary rotation centre, wherein the bars are able to rotate around the primary centre of rotation and around this new common centre, could be increased by varying the point of application of the forces that currently intervene by pushing against the same side as the thrust articulation.

This is achieved by means of a system for the adjustment and tensioning of the fixing straps of an orthopedic brace designed for rehabilitation of joints of the human body, whose features are described in the main claim.

The dependent claims of the solution according to this invention describe advantageous embodiments of the invention.

The main advantages of this solution are numerous and concern on one hand the possibility of carrying out adjustments on the lateral thrusts without intervening on the thrust mechanism but merely by means of the fixing straps that can be moved on the uprights located on the opposite side with respect to the mechanism, with much greater efficacy, and on the other hand the possibility of avoiding the presence of traditional mechanisms on the same side as the thrust cushion pad, which must be subjected to greater stress due to their proximity to the thrust centres of gravity.

The invention substantially foresees the use of active straps that are fitted on supports that slide with controlled movement on the bars of one of the two articulated couplings, in particular on the bars opposite the coupling provided with the thrust cushion pad.

This system according to the invention foresees that the knee brace is put on the body with the "active" straps in a horizontal and slightly pre-tautened position, and the sliding guides are then moved along the axes of one of the two articulated couplings.

The ends of each strap are attached to these sliding guides in order to generate a force "F", after the tautening of the straps, which presses the articulated coupling, opposite the coupling with the sliding guides, towards the inside of the knee brace.

Thanks to the central pin, this articulated coupling collapses inwards, allowing all the force "F" to press against the side of the knee (condyle) in order to straighten a joint impaired by varism or valgism.

The same system can be applied to orthoses for other joints of the human body.

### DESCRIPTION OF THE DRAWINGS

Other features and advantages of the invention will become clear on reading the description given below of one embodiment, provided as a non-limiting example, with the help of the accompanying drawings, in which:
- figures 1 to 3 represent schematic views of an orthosis according to the invention with the straps positioned respectively in a pre-tautening condition, a controlled traction condition and a blocked condition in the controlled traction position;
- figures 4 and 5 show schematic and prospective views of an orthosis provided with strap adjustment means according to the invention in a first and second tautening condition;
- figures 6 and 7 are schematic views showing the rear and front parts of a sliding guide with controlled movement on the respective upright and in a released working condition that allows its movement;
- figure 8 is a front schematic view with the pin forming part of the cursor shown in both the blocked position and in the release position;
- figure 9 shows a cross-section view along the line A-A of figure 8 of the sliding guide on the respective bar;
- figures 10 and 11 are schematic views showing the rear and front parts of a sliding guide with controlled movement on the respective upright and in a working blocked condition that ensures its blocking in the required position;
- figure 12 is a front schematic view;
- figure 13 is a cross section view along the line B-B of figure 12;
- figure 14 is a schematic view showing a possible way of controlling the movements of the sliding guide on the respective bars.

### DESCRIPTION OF AN EMBODIMENT OF THE INVENTION

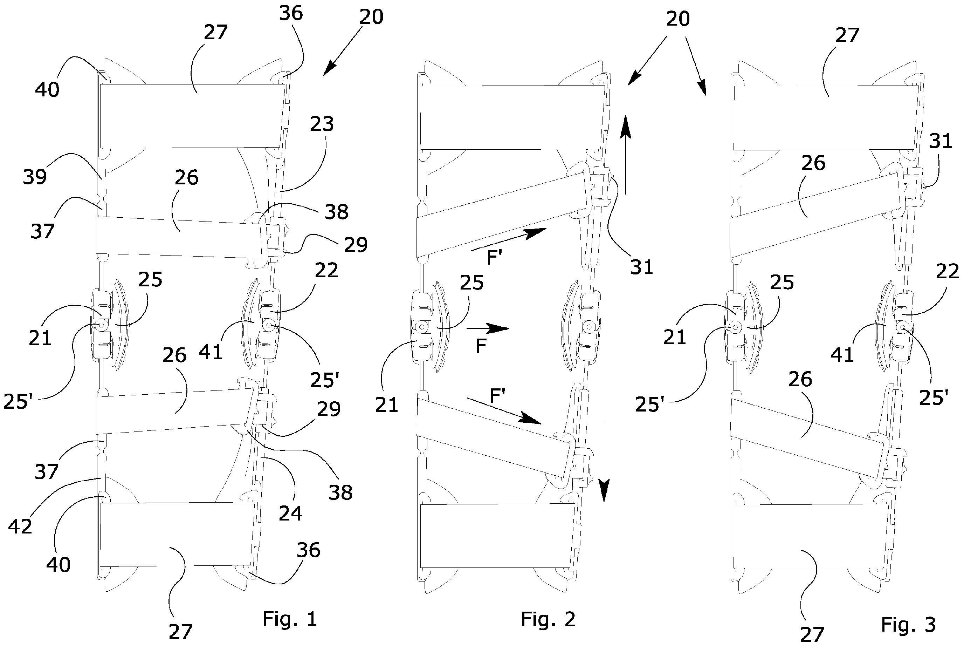

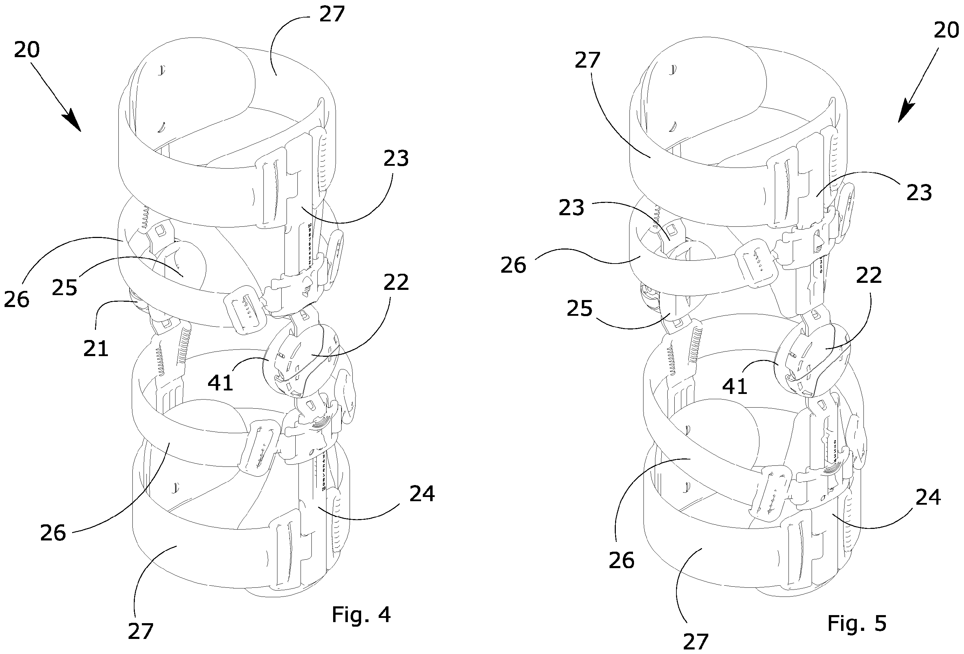

Referring first of all to figures 1 to 5, the numeral 20 denotes in its entirety an orthosis which, in the case shown here, is designed for the knee, bearing in mind that the system described below can also be applied to other orthoses for joints, such as for the elbow, the ankle, and others.

The orthosis 20 consists of a pair of articulated couplings 21 and 22, only one of which is the biaxial multicentre type, in this case the coupling 21, with controlled angular excursion, wherein each coupling acts as a support for relative pairs of femoral bars, respectively bars 23, 24 for the coupling 22 and bars 39, 42 for the coupling 21.

In the illustrated example, each of the couplings 21 and 22 comprises a platform, respectively 25 and 41, consisting of a cushion pad to support the part of the body to be treated, such as for example the condylar sectors of the knee, the elbow, or other joint, and is designed in such a way as to obtain two primary centres of rotation on the plane by the pins of the bars which rotate with respect to each other on common horizontal axes, and for just one coupling, which in the case described consists of the coupling 21, a secondary single-centre system defined by the pin 25', visible in figures 1 to 3, which eliminates the restraint of flatness of the bars 39 and 42, in such a way that, by using a lateral thrust, the coupling can bend angularly towards the inside of the orthoses, actually separating itself from the original primary centre of rotation.

The secondary centre of rotation 25' of the coupling 21 frees the bars 39 and 42 from merely rotating on their own plane, and makes it possible to apply lateral thrusts on the joint which are useful in axial correction, such as in valgus and varus deformities or when it is necessary to carefully monitor joints which by nature develop a varus or valgus angle, such as for example the elbow.

The couplings and the bars form the two side support sectors which are placed around and restrained on the joint by means of straps 26 and 27, the first closer to the coupling and the second farther away.

The straps 26 and 27 are attached to rings known as "loops" which are usually round or rectangular, with an elongated slot appropriate for the size of the straps, and which hold the straps on the brace and allow the orthosis to be blocked in place on the joint to be treated. More specifically, the straps 26 pass through the loops 37 relative to the coupling 21 and 38 relative to the coupling 22. The straps 27 pass through the loops 40 relative to the coupling 21 and 36 relative to the coupling 22. The straps are made, in fact, from elastic or rigid or mixed material and present sectors with Velcro strips or fixable in another way, such as by buckles or similar, in order to block the orthosis in the necessary position, that is with the couplings close to the axis of the joint.

According to the invention, the loops 38, designed to fix the straps 26 on the bars 23 and 24 of the coupling 22, are positioned on the opposite side with respect to the thrust condylar cushion pad 25, and form part, as an additional item or an integral part, of guides 29 which slide along the same femoral 23 and tibial 24 bars that allow the mobile bracket to be moved from a proximal to a distal position, with respect to the centre of the coupling 41, along the bar on which the guide is attached.

In other words, the straps 26 that pass through the loops 38, positioned on or forming part of the mobile guides 29, are "active" since they are subject to tautening caused by the movement of the loops on the guides.

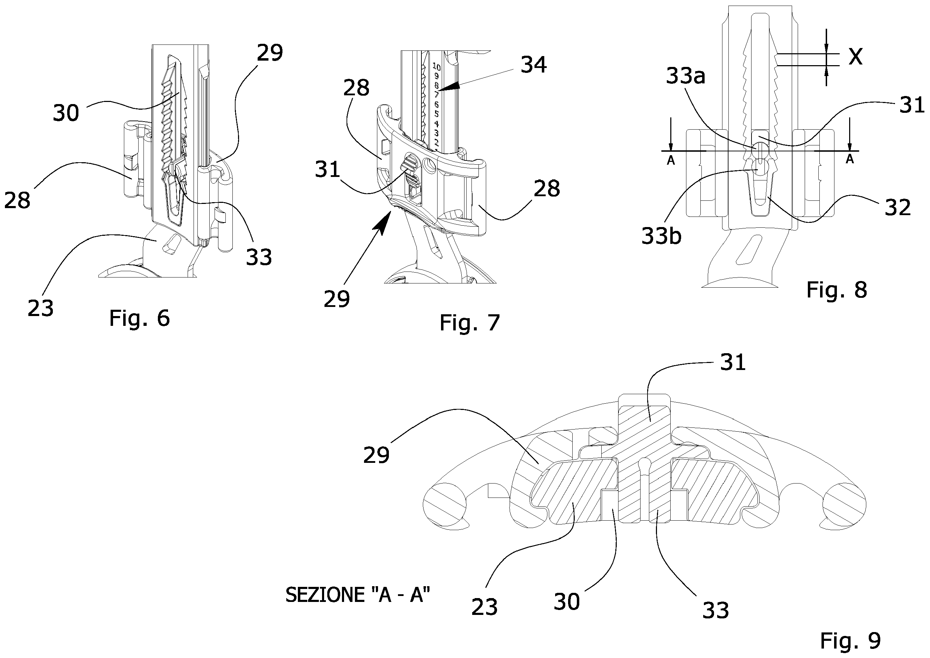

According to a preferred embodiment shown in figures 6 and 7, the bars 23 and 24 are made from metallic material on which a shaped plastic coating is applied, to form an assembly comprising a longitudinal slot 30 along their center-line and designed to accommodate a blocking cursor 31 and toothed segments 32 forming part of the guide 29.

The longitudinal slot 30 comprises internal toothed edges, with the reciprocal projections facing each other, allowing the guide 29 to be moved to the required position and then blocked there.

In fact, the guide 29 comprises two toothed segments 32 which interface with the toothing of the longitudinal slot 30 and flex inwards during the movement of the guide 29, thereby generating a snap-type movement.

This snap-type movement places the guide 29 in set positions and only in these positions can it be blocked thanks to the cursor 31.

The correct position is a direct consequence of the tension to be exerted, and only at this point can the guide 29 be blocked by a pin 33, forming part of the cursor 31, which maintains the teeth of the toothed segments in the position reached among those present in the toothing of the longitudinal slot 30.

As can be seen in figure 8, the pin 33 is moved by the cursor 31 from a release position 33a to a blocked position 33b of the guide 29 with respect to the bar on which it moves.

The exterior of the bars equipped with a strap-support sliding guide is also provided with a graduated scale 34 with increasing values, indicating a greater or lesser level of thrust exerted on the condylar cushion pad located on the opposite side of the orthosis.

The guide 29 is positioned along the longitudinal slot 30, according to set X value positions, as shown in figure 8. The X values, that is to say the length of the infeed of the guide along the bar, can be a fixed value or vary along the length of the slot in order to produce linear tautening increase curves or other types.

A particular feature of the system described above is due to the fact that the movement of the fixing bracket on the guide causes the traction of the articulated coupling on the opposite side, allowing the thrust of the cushion pad on the appropriate condyles of the joint.

This system allows the knee brace to be worn with the straps remaining "active", passing from a first step in which they are in a horizontal position and slightly pre-tautened (fig. 1) to a second step in which the guides 29 are moved (figs. 2 and 3) along the bars of the coupling 22, causing the coupling 21 on the opposite side to be pushed inwards.

Since the ends of the straps are attached to the guides 29, two forces F' are generated on the straps (fig. 2) when they are tautened, causing the inward movement of the coupling 21, opposite the coupling 22 forming part of the bars and guides, by a force F.

Thanks to the central pin 25', the coupling 21 collapses internally, allowing the entire force "F" to press against the side of the knee, at the level of the relative condyle, in order to straighten a joint impaired by varism or valgism.

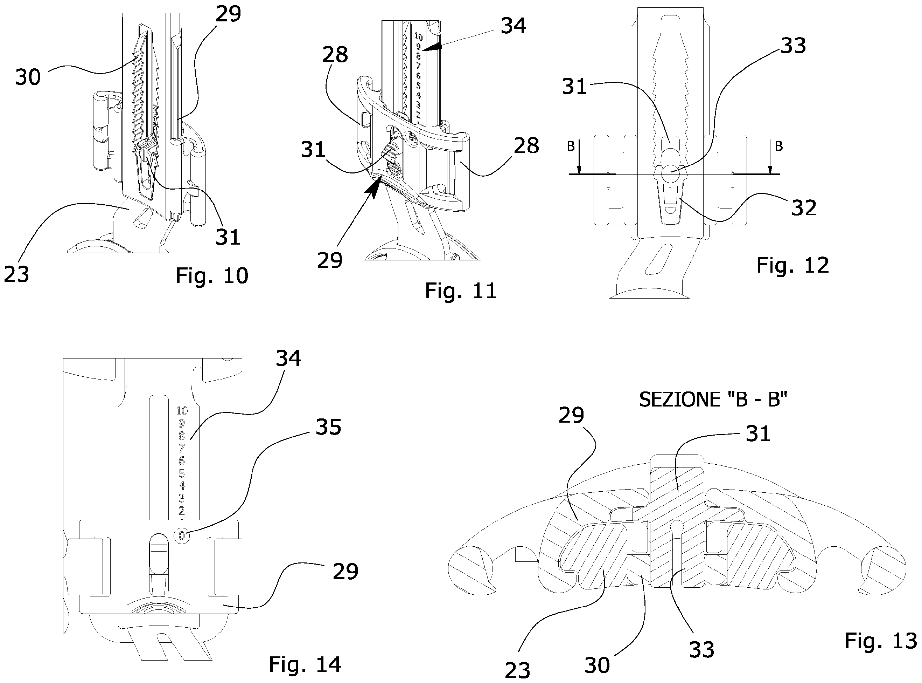

As can be seen in figure 14, the guide 29 can be provided with a system for optical reading of the graduated scale 34, consisting of a window 35 which makes the thrust level readable.

According to other embodiments, the means for blocking the guide 29 on the respective bar consist of a knob internally equipped with a cam which intercepts the toothed edges of the slot 30.

The means for blocking the guide 29 on the respective bar may also consist of any device which intercepts the toothed edges of the slot 30, that is to say a braking device that intercepts the bar on which the guide moves.

The invention as described above refers to a preferred embodiment. It is nevertheless clear that the invention is susceptible to numerous variations falling within the scope of the disclosure, in the context of technical equivalents.

The above description refers to an embodiment of a brace for the knee, but the same system can be applied to orthoses for other joints of the human.

## Claims

1. An orthosis (20) designed for rehabilitation of joints of the human body, said orthosis comprising fixing straps (26, 27) and a system for the adjustment and tautening of said fixing straps of said orthosis (20), this orthosis (20) further comprising articulated couplings (21, 22) with controllable angular excursion, one of which can move closer to or farther away from the centre of the joint along an axis parallel to the rotation axis of the articulated coupling, and in which each of the articulated couplings (21, 22) acts as a support for respective pairs of bars (39, 42 and 23, 24) and comprises respective platforms (25, 41), facing inwards, consisting of a cushion pad for the condylar sectors of the joint, and in which said bars constrained to the articulated couplings form lateral support sectors which are adapted to be attached to and fixed on the joint by means of said fixing straps (26, 27) in turn attached to feedthrough loops (36, 37, 38, 40), wherein these loops (38) accommodate the straps (26) and are present on the side of the orthosis opposite to a thrust platform (25), the system **characterised in that** these loops (38) are positioned on guides (29) sliding on respective bars (23, 24), and can be moved from a proximal to a distal position along the bar to which the guide is attached, and **in that** the bars (23, 24) comprise a longitudinal slot (30), designed to accommodate blocking means (31) associated with the guide (29).

2. The orthosis according to claim 1, **characterised in that** the blocking means consist of a cursor (31).

3. The orthosis according to claim 2, **characterised in that** the longitudinal slot (30) comprises inner toothed edges that can be intercepted by toothed segments (32) on the guide (29) and engaging with the cursor (31) allowing it to be positioned in one of the pre-determined points of the toothing of the longitudinal slot.

4. The orthosis according to claims 2 or 3 **characterised in that** the cursor (31) comprises a pin (33) which is interposed between the toothed segments (32), preventing disengagement with respect to the toothed slot (30) in the previously intercepted position, so that the sliding guide (29) is stopped in the position reached.

5. The orthosis according to claim 4, **characterised in that** the pin (33) is adapted to be moved by the locking cursor (31) from a release position (33a) in which the guide (29) is free to move along the respective bar to a blocking position (33b) in which the guide (29) is locked on the bar.

6. The orthosis according to any of the previous claims, **characterised in that** the visible side of the bars (23, 24) equipped with the sliding guide supporting the straps is provided with a graduated scale (34) with increasing values that correspond to the increasing thrust force (F) exerted by the condylar cushion pad (25), this force (F) being the result of the increasing traction (F') exerted by the straps (26) on the cushion pad.

7. The orthosis according to any of the previous claims, **characterised in that** the movement of the sliding guides (29) along the respective bars, in use, tautens the straps (26) which exert inward traction (F') on the articulated coupling on the opposite side, creating thrust force (F) of the cushion pads (25) on the condyles involved in the joint.

8. The orthosis according to any of the previous claims, **characterised in that** the articulated coupling (21) is equipped with a central pin (25') which allows flexion of the articulated coupling towards the interior or the exterior of the orthosis.

9. The orthosis according to claim 1, **characterised in that** the means for blocking the sliding guide (29) on the respective bar consist of a rotating knob connected internally to a cam that intercepts the toothed edges of the slot (30).

10. The orthosis according to claim 1, **characterised in that** the means for blocking the sliding guide (29) on the respective bar consist of a braking device that intercepts the bar on which the guide slides.

## Patentansprüche

1. Orthese (20), die zur Rehabilitation von Gelenken des menschlichen Körpers bestimmt ist, wobei die Orthese Befestigungslaschen (26, 27) und ein System für die Einstellung und Straffung der Befestigungslaschen der Orthese (20) umfasst,
wobei diese Orthese (20) ferner Gelenkverbindungen (21, 22) mit kontrollierbarer Winkelauslenkung umfasst, von denen eine sich näher an die oder weiter weg von der Mitte des Gelenks entlang einer Achse bewegen kann, die parallel zur Rotationsachse der Gelenkverbindung ist, und bei der jede der Gelenkverbindungen (21, 22) als Stütze für entsprechende Paare von Stäben (39, 42 und 23, 24) fungiert und entsprechende Plattformen (25, 41) umfasst, die nach innen weisen, bestehend aus einer Abfederungsstütze für die kondylären Gelenkbereiche, und wobei die Stäbe, die auf die Gelenkverbindungen beschränkt sind, seitliche Stützbereiche bilden, die dafür ausgelegt sind, am Gelenk mittels der Befestigungslaschen (26, 27) befestigt und auf dem Gelenk festgelegt sind, welche wiederum an Durchführungsschlaufen (36, 37, 38, 40) befestigt sind, wobei diese Schlaufen (38) die Laschen (26) aufnehmen und auf der Seite der Orthese gegenüber einer Schubplattform (25) vorhanden sind, wobei das System **dadurch gekennzeichnet ist,**
**dass**
diese Schlaufen (38) auf Führungen (29) positioniert sind, die auf entsprechenden Stäben (23, 24) gleiten und von einer proximalen zu einer distalen Position entlang des Stabes bewegt werden können, an dem die Führung befestigt ist, und dadurch, dass die Stäbe (23, 24) einen Längsschlitz (30) umfassen, der dafür bestimmt ist, Blockiermittel (31) aufzunehmen, die mit der Führung (29) verbunden sind.

2. Orthese nach Anspruch 1, **dadurch gekennzeichnet, dass** die Blockiermittel aus einem Schieber (31) bestehen.

3. Orthese nach Anspruch 2, **dadurch gekennzeichnet, dass** der Längsschlitz (30) innere gezähnte Kanten umfasst, die durch gezähnte Abschnitte (32) auf der Führung (29) abgefangen werden können und die in den Schieber (31) eingreifen, was ermöglicht, dass er in einem der vorgegebenen Punkte der Zähnung des Längsschlitzes positioniert werden kann.

4. Orthese nach Anspruch 2 oder 3, **dadurch gekennzeichnet, dass** der Schieber (31) einen Stift (33) umfasst, der zwischen den gezähnten Segmenten (32) eingefügt ist, was die Loslösung in Bezug auf den gezähnten Schlitz (30) in der vorherigen abgefangenen Position verhindert, sodass die Gleitführung (29) in der erreichten Position stoppt.

5. Orthese nach Anspruch 4, **dadurch gekennzeichnet, dass** der Stift (33) dafür ausgelegt ist, durch den Verriegelungsschieber (31) von einer Entriegelungsposition (33a), in der die Führung (29) sich frei entlang des entsprechenden Stabes bewegen kann, in eine Blockierstellung (33b) bewegt zu werden, in der die Führung (29) durch den Stab blockiert ist.

6. Orthese nach einem der vorherigen Ansprüche, **dadurch gekennzeichnet, dass** die sichtbare Seite der Stäbe (23, 24), die mit der Gleitführung versehen ist, welche die Laschen stützt, mit einer graduierten Skala (34) mit steigenden Werten versehen ist, die der ansteigenden Schubkraft (F) entsprechen, welche auf die kondyläre Abfederungsstütze (25) ausgeübt wird, wobei diese Kraft (F) das Ergebnis des wachsenden Zugs (F') ist, der auf die Laschen (26) auf der Abfederungsstütze ausgeübt wird.

7. Orthese nach einem der vorherigen Ansprüche, **dadurch gekennzeichnet, dass** die Bewegung der Gleitführungen (29) entlang der entsprechenden Stäbe bei der Verwendung die Laschen (26) strafft, die einen Zug nach innen (F') auf die Gelenkverbindung auf der entgegengesetzten Seite ausüben, was eine Schubkraft (F) der Abfederungsstützen (25) auf den Kondylen erzeugt, die am Gelenk beteiligt sind.

8. Orthese nach einem der vorherigen Ansprüche, **dadurch gekennzeichnet, dass** die Gelenkverbindung (21) mit einem zentralen Stift (25') ausgerüstet ist, der eine Beugung der Gelenkverbindung zur Innenseite oder Außenseite der Orthese ermöglicht.

9. Orthese nach Anspruch 1, **dadurch gekennzeichnet, dass** die Mittel zum Blockieren der Gleitführung (29) auf dem entsprechenden Stab aus einem rotierenden Knopf besteht, der intern mit einem Nocken verbunden ist, welcher die gezähnten Kanten des Schlitzes (30) abfängt.

10. Orthese nach Anspruch 1, **dadurch gekennzeichnet, dass** das Mittel zum Blockieren der Gleitführung (29) auf dem entsprechenden Stab aus einer Bremsvorrichtung besteht, die den Stab abfängt, auf dem die Führung gleitet.

## Revendications

1. Orthèse (20) conçue pour la rééducation d'articulations du corps humain, ladite orthèse comprenant des sangles de fixation (26, 27) et un système de réglage et de tension desdites sangles de fixation de ladite orthèse (20),
cette orthèse (20) comprenant en outre des accouplements articulés (21, 22) à excursion angulaire commandée, dont l'un peut se rapprocher ou s'éloigner du centre de l'articulation le long d'un axe parallèle à l'axe de rotation de l'accouplement articulé, et dans laquelle chacun des accouplements articulés (21, 22) servent de support pour des paires de barres respectives (39, 42 et 23, 24) et comprennent des plates-formes respectives (25, 41), tournées vers l'intérieur, constituées d'un coussinet destiné aux secteurs condyliens de l'articulation, et dans laquelle lesdites barres contraintes vers les accouplements articulés forment des secteurs de support latéraux qui sont adaptés pour être attachés et fixés à l'articulation par le biais desdites sangles de fixation (26, 27) attachés à leur tour à des boucles de passage (36, 37, 38, 40), dans laquelle ces boucles (26) reçoivent les sangles (28) et sont présentes du côté de l'orthèse qui est opposé à une plateforme de poussée (25), le système est **caractérisé en ce que** ces boucles (38) sont positionnées sur des guides (29) coulissant sur des barres respectives (23, 24), et peuvent être déplacées d'une position proximale à une position distale le long de la barre à laquelle le guide est attaché, et **en ce que** les barres (23, 24) comprennent une fente longitudinale (30) conçue pour recevoir des moyens de blocage (31) associés au guide (29).

2. Orthèse selon la revendication 1, **caractérisée en ce que** les moyens de blocage consistent en un curseur (31).

3. Orthèse selon la revendication 2, **caractérisée en ce que** la fente longitudinale (30) comporte des bords intérieurs dentés pouvant être interceptés par des segments dentés (32) situés sur le guide (29) et venant s'engager avec le curseur (31) pour le positionner dans l'un des points prédéterminés de la denture de la fente longitudinale.

4. Orthèse selon la revendication 2 ou 3, **caractérisée en ce que** le curseur (31) comprend une tige (33) qui est interposée entre les segments dentés (32), de manière à empêcher le désengagement de la fente dentée (30) dans la position interceptée précédemment, de sorte que le guide coulissant (29) soit arrêté dans la position atteinte.

5. Orthèse selon la revendication 4, **caractérisée en ce que** la tige (33) est adaptée pour être déplacée par le curseur de blocage (31) d'une position de libération (33a), dans laquelle le guide est libre de se déplacer le long de la barre respective, à une position de blocage (33b) dans laquelle le guide (29) est bloqué sur la barre.

6. Orthèse selon l'une quelconque des revendications précédentes, **caractérisée en ce que** le côté visible des barres (23, 24) équipées du guide coulissant supportant les sangles est muni d'une échelle graduée (34) comportant des valeurs croissantes correspondantes à la force de poussée croissante (F) exercée par les coussins condyliens (25), cette force (F) résultant de la traction croissante (F') exercée par les sangles (26) sur le coussin.

7. Orthèse selon l'une quelconque des revendications précédentes, **caractérisée en ce que** le mouvement des guides coulissants (29) le long des barres respectives tend, en utilisation, les sangles (26) qui exercent une traction vers l'intérieur (F') sur l'accouplement articulé du côté opposé, créant une force de poussée (F) des coussins (25) sur les condyles impliqués dans l'articulation.

8. Orthèse selon l'une quelconque des revendications précédentes, **caractérisée en ce que** l'accouplement articulé (21) est équipé d'une tige centrale (25') permettant la flexion de l'accouplement articulé vers l'intérieur ou l'extérieur de l'orthèse.

9. Orthèse selon la revendication 1, **caractérisée en ce que** les moyens de blocage du guide coulissant (29) sur la barre respective consistent en un bouton rotatif raccordé à l'intérieur à une came qui intercepte les bords dentés de la fente (30).

10. Orthèse selon la revendication 1, **caractérisée en ce que** les moyens de blocage du guide coulissant (29) sur la barre respective consistent en un dispositif de freinage qui intercepte la barre sur laquelle le guide coulisse.
